# EUROPÄISCHE PATENTSCHRIFT

(11) **EP 3 743 127 B1**
(45) Veröffentlichungstag und Bekanntmachung des Hinweises auf die Patenterteilung: **30.07.2025**
(21) Anmeldenummer: 19704738.4
(22) Anmeldetag: 24.01.2019
(51) Int. Cl.: A61M 1/00, A61M 3/02, A61L 2/14, A61K 33/00, A61K 45/06, A61L 15/18, A61L 15/42, A61F 13/05, A61K 33/44, A61L 2/00, A61N 5/06

(54) **KOMBINATION AUS WUNDSPÜLLÖSUNG UND KALTPLASMA ZUR WUNDBEHANDLUNG**
COMBINATION OF A WOUND-RINSING SOLUTION AND COLD PLASMA FOR THE TREATMENT OF WOUNDS
COMBINAISON DE SOLUTION DE RINÇAGE DE PLAIES ET DE PLASMA FROID TRAITER DES PLAIES

(30) Priorität: 26.01.2018 DE 102018101748
(43) Veröffentlichungstag der Anmeldung: 02.12.2020
(73) Patentinhaber: Westfälische Wilhelms-Universität Münster, 48149 Münster (DE)
(72) Erfinder: ROTERING, Heinrich, 48329 Havixbeck (DE)
(74) Vertreter: Schiweck Weinzierl Koch Patentanwälte Partnerschaft mbB
(86) Internationale Anmeldenummer: PCT/EP2019/051680
(87) Internationale Veröffentlichungsnummer: WO 2019/145383

(56) Entgegenhaltungen:
- EP-A1- 2 206 521
- DE-A1- 102014 002 000
- DE-A1- 102017 106 482
- US-A1- 2012 046 602
- US-A1- 2015 320 604
- US-B1- 8 267 884
- EMMERT S ET AL: "Treatment of chronic venous leg ulcers with a hand-held DBD plasma generator", PLASMA MEDICINE, BEGELL HOUSE, INC, US, vol. 2, no. 1-3, 1 January 2012 (2012-01-01), pages 19 - 32, XP009512719, ISSN: 1947-5764, DOI: 10.1615/PLASMAMED.2013005914

## Beschreibung

Die vorliegende Erfindung betrifft die Kombination von Wundspüllösung, kaltem atmosphärischem Plasma und einem Aktivkohleverband zur Verwendung in einem Verfahren bei der Behandlung von Wunden. Die vorliegende Offenbarung betrifft ferner ein Verfahren zur Wundbehandlung, umfassend das Spülen der Wunde mit einer Wundspüllösung und das Behandeln der Wunde mit kaltem atmosphärischem Plasma.

Viele Wunden, insbesondere chronische Wunden, verheilen nur sehr langsam oder gar nicht. Häufig sind hierfür schwere Infektionen verantwortlich, welche oft durch Bakterien verursacht werden, welche aufgrund von Resistenzen nicht mehr oder kaum noch antibiotisch bekämpft werden können.

Große, von außen zugängliche und oft chronische Wunden werden unter anderem mit Wundspüllösungen zur Reinigung der Wunde behandelt. Dies zeigt US2015/320604, indem die Verwendung einer Wundspullösung zur Behandlung von Wunden, wobei ferner ein Unterdruck-Aktivkohleverband verwendet wird, offenbart wird. US8267884 umfasst ebenfalls ein Verfahren zur Wundbehandlung, das das Spülen der Wunde mit einer Wundspüllösung, und das Behandeln der Wunde mit kaltem atmosphärischem Plasma umfasst.

Zum einen werden mit Wundspüllösungen Gewebereste, Nekrosepartikel, überschüssiges Exsudat, Belag, Verbandsrückstände, Zelltrümmer, etc. entfernt, ebenso werden so Biofilme - oft zusammen mit Antiseptika und chirurgischem Debridement - beseitigt.

Solche Wundspüllösungen sind in der Regel isotonisch (physiologisch) und können verschiedene Antiseptika enthalten, welche - je nach Einsatzort und Infektionsursache - bakterizide, bakteriostatische, fungizide, fungistatische, und/oder viruzide Wirkstoffe umfassen. Einige Wundspüllösungen umfassen auch reaktive Sauerstoffe wie Singulett-Sauerstoff, welcher ggf. zunächst komplexiert (bspw. Natriumhypochlorit) vorliegt und anschließend freigesetzt wird (Kammerlander et al., pro care 08/2012, S. 26-29). Jedoch sind nicht alle Wunden durch Wundspüllösungen heilbar, oft gibt es nach Behandlung Rezidive.

Die vorliegende Erfindung adressiert dieses technische Problem, wie in der vorliegenden Beschreibung ausgeführt und in den Ansprüchen definiert.
**Figur 1A** zeigt den in Beispiel 4 beschriebenen Patienten vor der erfindungsgemäßen Wundbehandlung. Der Patient hatte eine infizierte Driveline.
**Figur 1B** zeigt eine PET-CT Aufnahme des in Beispiel 4 beschriebenen Patienten vor der erfindungsgemäßen Wundbehandlung. Der Patient hatte eine infizierte Driveline.
**Figur 1C** zeigt den in Beispiel 4 beschriebenen Patienten nach 3 Wochen Behandlungszeit mit der erfindungsgemäßen Wundbehandlung.
**Figur 2** zeigt eine Driveline mit Aktivkohle-NPWT Verband: Aktivkohlefolie (1) wurde im Wundgrund platziert, umhüllt die Driveline und ragt aus der Wunde heraus, um das umliegende Hautareal zu überdecken; ein grau-schwarzer NPWT Schwamm (2) wird über die Aktivkohlefolie eingebracht.
**Figur 3** zeigt den Wechsel von Spülung und Kaltplasma (CAP) sowie die Versiegelung der Wunde mit Aktivkohle-NPWT.

Wie im Rahmen der vorliegenden Erfindung überraschend herausgefunden wurde, eignet sich eine Kombination von Wundspüllösung, kaltem atmosphärischem Plasma (im Folgenden auch "Kaltplasma" oder "CAP" genannt) und einem Aktivkohleverband sehr gut zur Verwendung in der Wundbehandlung. Wie gezeigt werden konnte, können somit chronische Wunden weitestgehend saniert und auch die Zahl bzw. Intensität an Rezidiven deutlich verringert werden. Unerwünschte Nebenwirkungen wurden nicht beobachtet. Es stellte sich heraus, dass eine Behandlung von Wunden mit Wundspüllösung alleine, z.B. ActiMaris^{®} zu keinem zufriedenstellenden Erfolg führte, wohingegen die Kombination von Wundspüllösung, Kaltplasma und einem Aktivkohleverband zu mehr als zufriedenstellenden, im vorliegenden Fall gar überraschenden, Therapieerfolgen bei der Wundbehandlung führte.

Die vorliegende Erfindung betrifft daher die Kombination von Wundspüllösung, kaltem atmosphärischem Plasma und einem Aktivkohleverband in einem Verfahren bei der Behandlung von Wunden, umfassend folgende Schritte:
(a) Spülen der Wunde mit einer Wundspüllösung,
(b) Behandeln der Wunde mit kaltem atmosphärischem Plasma, und
(c) Anlegen eines Unterdruck-Aktivkohleverbandes auf die Wunde.

Die vorliegende Erfindung ist in den beigefügten Ansprüchen genauer dargelegt.

Alternativ betrifft die vorliegende Offenbarung die Verwendung einer Kombination von Wundspüllösung und kaltem atmosphärischem Plasma zur Anwendung bei der Behandlung von Wunden.

In einer Ausführungsform die vorliegende Offenbarung betreffend wird darüber hinaus auch ein Aktivkohleverband zur Wundbehandlung verwendet, bevorzugt ein Unterdruck-Aktivkohleverband. Beispiele für geeignete Aktivkohleverbände sind dem Fachmann bekannt und umfassen u.a. Actisorb^{®}, Askina^{®} Carbosorb, CarboFlex^{®}, Vliwaktiv^{®} AG Saugkompresse, und andere. Als Unterdruck-Aktivkohleverband kann u.a. ein Verband aus Aktivkohle und Unterdruckschwamm verwendet werden, wie dem Fachmann bspw. als "advanced Negative Pressure Wound Therapy (aNPWT) oder für den deutschsprachigen Raum auch als Aktivkohle-NPWT bekannt ist. Bei der Verwendung eines solchen Aktivkohle-NPWT (aNPWT) ist eine intensive Wundtherapie mit kürzeren Wechselzeiten (alle 2-3 Tage) möglich, da die Kohlefolie für eine aktive Reinigung sorgt. Die Freisetzung von Sauerstoffradikalen ist bei dieser Verwendung ausdrücklich erwünscht.

Die aNPWT Behandlungsmethode ist allerdings von dem NPWT Standardverfahren aus dem Stand der Technik zu unterscheiden z.B. Avance^{®}Flex (Mölnlycke^{®}). Hierbei handelt es sich um die Verwendung eines grünen Schwammes, anhand dessen die Detektion von Blutverlust durch die Verfärbung des Schwammes möglich ist. Bei diesem Verfahren von Mölnlycke Health Care wird ausdrücklich von der Verwendung von Hypochloridlösung sowie Wasserstoffperoxid aufgrund der Freisetzung von Sauerstoffradikalen abgeraten. Es handelt sich lediglich um ein modifiziertes NPWT-Verfahren (die Verwendung eines grünen Schwamms), jedoch ohne Aktivkohleverband im Sinne der Wundreinigung wie bei der aNPWT verwendet.

Bei der Verwendung eines Aktivkohle-NPWT kann ferner die Aktivkohle unter dem Unterdruckschwamm residuale Keime fixieren und somit eine erneute Progression der Infektion reduzieren. Damit kann erfindungsgemäß der Behandlungsfortschritt durch die Kombination aus Wundspüllösung und kaltem atmosphärischem Plasma weiter unterstützt werden. Besonders bevorzugt ist bei der erfindungsgemäßen Anwendung ein Aktivkohleverband, bei dem der Aktivkohle enthaltende Teil direkt auf die Wunde aufgebracht (d.h. gelegt) wird. Dadurch können Bakterien ohne Barriere gebunden werden. Ein bevorzugter Aktivkohleverband, der ein direktes Aufbringen des Aktivkohle enthaltenden Teils des Verbands ermöglicht ist Zorflex^{®} Aktivkohle (Chemviron (England)). Bislang hat noch keine Firma die Kohlefolie im direkten Kontakt mit dem Wundgrund empfohlen.

Die vorliegende Offenbarung betrifft ferner ein Verfahren zur Wundbehandlung, umfassend
(a) Spülen der Wunde mit einer Wundlösung, und
(b) Behandeln der Wunde mit kaltem atmosphärischem Plasma.

Bevorzugt verbleibt im Wundbett ein feuchter Film der Spüllösung ehe die Wunde mit kaltem atmosphärischem Plasma behandelt wird.

In einer Ausführungsform die vorliegende Offenbarung betreffend kann das Verfahren auch noch einen weiteren Schritt
(c) Erneutes Spülen der Wunde mit einer Wundspüllösung (bevorzugt mit der gleichen Wundspüllösung wie in Schritt (a)) und/oder
(d) Anlegen eines Aktivkohleverbandes auf die Wunde.
umfassen. Die Schritte (a), (b) sowie ggf. (c) und/oder (d) erfolgen erfindungsgemäß bevorzugt in der Reihenfolge (a), (b), (c), (d).

Das offenbarte Verfahren kann ferner einen weiteren Schritt des Behandelns der Wundspüllösung mit kaltem atmosphärischem Plasma umfassen, bevorzugt vor Schritt (a) und/oder vor Schritt (c).

Auch im Zusammenhang für Schritt (d) handelt es sich erfindungsgemäß bevorzugt um einen Unterdruck-Aktivkohleverband. Beispiele für geeignete (Unterdruck-) Aktivkohleverbände sind dem Fachmann bekannt wie bereits oben dargelegt.

Wie oben bereits ausgeführt betrifft die vorliegende Erfindung eine Kombination von Wundspüllösung, kaltem atmosphärischem Plasma und einem Aktivkohleverband zur Verwendung in einem Verfahren bei der Behandlung von Wunden, umfassend:
(a) Spülen der Wunde mit einer Wundspüllösung,
(b) Behandeln der Wunde mit kaltem atmosphärischem Plasma, und
(c) Anlegen eines Unterdruck-Aktivkohleverbandes auf die Wunde.

Bevorzugt verbleibt im Wundbett ein feuchter Film der Spüllösung ehe die Wunde mit kaltem atmosphärischem Plasma behandelt wird.

In einer Ausführungsform der vorliegenden Erfindung kann die vorstehend beschriebene Kombination von Wundspüllösung, kaltem atmosphärischem Plasma und einem Aktivkohleverband zur Verwendung in einem Verfahren bei der Behandlung von Wunden auch noch einen weiteren Schritt:
(d) Erneutes Spülen der Wunde mit einer Wundspüllösung (bevorzugt mit der gleichen Wundspüllösung wie in Schritt (a))
umfassen. Die Schritte (a), (b), (c) und ggf. (d) erfolgen erfindungsgemäß bevorzugt in der Reihenfolge (a), (b), (c), (d).

In einer Ausführungsform der vorliegenden Erfindung kann die vorstehend beschriebene Kombination von Wundspüllösung, kaltem atmosphärischem Plasma, und einem Aktivkohleverband zur Verwendung in einem Verfahren bei der Behandlung von Wunden ferner den weiteren Schritt des Behandelns der Wundspüllösung mit kaltem atmosphärischen Plasma umfassen, bevorzugt vor Schritt (a) und/oder vor Schritt (d).

Auch im Zusammenhang für Schritt (c) handelt es sich erfindungsgemäß um einen Unterdruck-Aktivkohleverband. Beispiele für geeignete (Unterdruck-) Aktivkohleverbände sind dem Fachmann bekannt wie bereits oben dargelegt.

Bevorzugt verbleibt im Wundbett ein feuchter Film der Spüllösung ehe die Wunde mit kaltem atmosphärischem Plasma behandelt wird.

Weiterhin betrifft die vorliegende Erfindung in einer Ausführungsform alternativ auch eine Kombination von Wundspüllösung, kaltem atmosphärischem Plasma und einem Aktivkohleverband zur Anwendung bei der Behandlung von Wunden, wobei die Wundspüllösung hergerichtet ist zur Spülung von Wunden und das kalte atmosphärische Plasma Injektion hergerichtet ist zur Behandlung von Wunden und ein Aktivkohleverband hergerichtet ist zum Anlegen auf die Wunde.

Wundspüllösungen im Zusammenhang mit der vorliegenden Erfindung umfassen insbesondere Lösungen, die zur Wundreinigung geeignet sind und können auf die zu spülende bzw. reinigende Wunde angepasst sein wie dem Fachmann bekannt. In einer Ausführungsform handelt es sich hierbei um Wundspüllösungen, die steril, schmerzfrei oder -arm, physiologisch, nicht reizend oder ätzend, und/oder auf mindestens 28 °C erwärmbar sind. Wundspüllösungen im Zusammenhang mit der vorliegenden Erfindung können u.a. Salze (z.B. NaCl, Meersalz) und/oder Elektrolyte (z.B. Natrium, Kalium, Kalzium), Alkohole (bspw. Ethanol, Isopropanol, Phenoxyethanol), quartäre Ammoniumverbindungen (bspw. Benzalkonium, Cetylpyridinium, Octenidin, Polyhexanid), Jod-haltige Verbindungen (bspw. Povidon-Jod/ PVP-Jod), Chlorhaltige Verbindungen (bspw. Chlorhexidin), Hexetidin, Bibrocathol, Taurolidin, Enzyme (bspw. Varidase, Fibrolan), und/oder (weitere) bakterizide, bakteriostatische, fungizide, fungistatische, und/oder viruzide Inhaltsstoffe (Antiseptika) enthalten. Solche bakteriziden, bakteriostatischen, fungiziden, fungistatischen, und/oder viruziden Inhaltsstoffe können bspw. umfassen: Antibiotika, reaktive Verbindungen wie ROS (Reactive Oxygen Species) und/oder RNS (Reactive Nitrogen Species). Beispiele für ROS in diesem Zusammenhang umfassen Singulett-Sauerstoff (¹O₂), Sauerstoffradikale, Wasserstoffperoxid (H₂O₂), Ozon (O3) und andere. Beispiele für RNS umfassen u.a. NO und NO₂. ROS als potentieller Bestandteil von Wundspüllösungen können erfindungsgemäß auch in anderen Verbindungen komplexiert sein, bspw. Singulett-Sauerstoff in Form von Natriumhypochlorit (NaOCl). Bei komplexiertem Singulett-Sauerstoff wie NaOCl wird der Singulett-Sauerstoff dann in der Regel bei Wundkontakt freigesetzt wie dem Fachmann bekannt. In einer Ausführungsform der vorliegenden Erfindung enthält die Wundspüllösung etwa 0,01 - 0,1 Gew% NaOCl, bevorzugt etwa 0,01 - 0,08, etwa 0,02 - 0,08, etwa 0,02 - 0,07, etwa 0,02 - 0,06 oder etwa 0,02 - 0,05 oder 0,03 - 0,05 Gew% NaOCl.

In einer Ausführungsform der vorliegenden Erfindung enthält die Wundspüllösung Singulett-Sauerstoff (¹O₂), wobei hier wie bereits dargestellt auch komplexierter Singulett-Sauerstoff (¹O₂) umfasst ist wie bspw. Natriumhypochlorit (NaOCl). Bei komplexiertem Singulett-Sauerstoff wie NaOCl wird der Singulett-Sauerstoff in der Regel bei Wundkontakt freigesetzt. In einer besonderen Ausführungsform enthält die Wundspüllösung ferner Salz, bspw. NaCl, und/oder Meersalz. In einer besonderen Ausführungsform liegt der Salzgehalt (bspw. NaCl-Gehalt) unter 5, bevorzugt 4 oder 3 Gew% der Wundspüllösung. In einer weiteren Ausführungsform der vorliegenden Erfindung enthält die Wundspüllösung Polyhexanid und/oder Octenidin, bevorzugt Polyhexanid. Geeignete Wundspüllösungen sind dem Fachmann bekannt und können auf die zu behandelnde Wunde abgestimmt sein. Im Rahmen der vorliegenden Erfindung kann es sich u.a. bei der Wundspüllösung bspw. um ActiMaris^{®}, Lavanox^{®}, Kerrasol^{®}, BIOsept^{®} Wundspüllösung, oder Microdacyn60^{®} Wound Care handeln, bevorzugt ActiMaris^{®}, Lavanox^{®} oder Kerrasol^{®}, und besonders bevorzugt Lavanox^{®} oder Kerrasol^{®}.

Bei den erfindungsgemäß zu behandelnden Wunden kann es sich grundsätzlich um alle Arten von Wunden handeln, welche einer Wundspüllösung und einer Behandlung mit Kaltplasma zugänglich sind. Bevorzugt handelt es sich dabei auf Wunden auf der Haut oder Schleimhaut, welche bevorzugt ohne operativen Eingriff zugänglich sind (oberflächliche Wunde). In einer Ausführungsform der vorliegenden Erfindung ist die zu behandelnde Wunde infiziert bzw. entzündet mit einem oder mehreren Bakterien und/oder Viren, bevorzugt mit Bakterien. Typische bakterielle Infektionen und Entzündungen können hervorgerufen sein durch bspw. *Enterobacter* sp. (bspw. *E. cloacae*)*, Pseudomonas* sp. (bspw. *P. aeroginosa*)*, Staphylococcus* sp. (bspw. *S. aureus*)*, Klebsiella* sp. (bspw. *K. pneumonia*)*, E. coli, Streptococcus* sp., und/oder *Proteus* sp. Die erfindungsgemäß zu behandelnde Wunde ist in einer Ausführungsform akut oder chronisch infiziert bzw. entzündet, bevorzugt chronisch (chronische Wunde). "Chronisch" in diesem Zusammenhang kann bedeuten, dass die Wunde innerhalb von mind. 4, 6 oder 8 Wochen nach Wundentstehung noch nicht abgeheilt ist, bevorzugt nach mind. 4 Wochen.

Im Zusammenhang mit der vorliegenden Erfindung wird die zu behandelnde Wunde unter Verwendung einer Kombination aus Wundspüllösung wie hier dargestellt und kaltem (auch "nicht-thermisch" genannt), atmosphärischem Plasma (hier auch "Kaltplasma" genannt) behandelt. Kaltes, atmosphärisches Plasma (Kaltplasma) umfasst dabei teilweise ionisiertes Gas (bspw. Umgebungsluft, Argon, Heliummischungen bevorzugt mit geringer Beimischung von Sauerstoff, oder Stickstoff; bevorzugt Argon), welches unter atmosphärischen Bedingungen (Umgebungsdruck) erzeugt wird. Die Temperatur des Plasmas übersteigt dabei bevorzugt nicht 50 °C, bevorzugt nicht 40 °C. In einer Ausführungsform liegen beim Kaltplasma nur bis zu 0,1 bis 10 ppb (parts per billion, Teilchen pro Milliarden) ionisierte Teilchen vor, bspw. 0,5 bis 5 ppb oder etwa 1 ppb. Beim Kaltplasma können im Rahmen der vorliegenden Erfindung Elektronen, Ionen, Radikale, ROS, RNS und/oder UV-Strahlung erzeugt werden. Erzeugung und Anwendung von Kaltplasma ist dem Fachmann bekannt wie bspw. angeboten von terraplasma medial GmbH (Deutschland; http://terraplasma-medical.com/). Typische Gase zur Plasmaerzeugung können erfindungsgemäß u.a. umfassen: Luft, Argon, Heliummischungen bevorzugt mit geringer Beimischung von Sauerstoff, und/oder Stickstoff, bevorzugt Argon.

Erfindungsgemäß geeignete Geräte zur Erzeugung und Anwendung von Kaltplasma und Anwendung in der medizinischen Behandlung sind dem Fachmann bekannt und umfassen bspw. PlasmaDerm^{®} (Cinogy), kINPen^{®} (INP Greifswald), Plasma One (plasma Medical Systems GmbH) und SteriPlas^{®} (Adtec Healthcare). Die Anwendung des Kaltplasmas kann dabei grundsätzlich auf jedwede geeignete Art geschehen wie dem Fachmann bekannt und wie bspw. beschrieben in Eswaramoorthy et al., Biophys (2017), 9: 895-917.

Wie bereits beschrieben, wird die Wunde im Zusammenhang mit der vorliegenden Erfindung zunächst mit einer geeigneten Wundspüllosung gespült. Die Einwirkzeit kann dabei abhängig von der vorliegenden Wunde variieren und beträgt typischerweise ca. 5 - 10 min, bevorzugt ca. 7 bis 8 min. Je nach Zugänglichkeit der Wunde kann die Wundspüllösung direkt oder auch mit Hilfsmitteln wie bspw. getränkten Kompressen aufgetragen werden. Ebenso können im Rahmen der vorliegenden Erfindung auch infizierte Bereiche in Wundnähe, die selbst nicht Teil der Wunde sind aber eine erneute Infektion der Wunde begünstigen könnten (bspw. Kabel, Schläuche, Implantate oder andere Geräte), mit der Wundspüllösung behandelt werden. Danach kann ggf. die Wundspüllösung mittels geeigneter Maßnahmen (bspw. Kompressen) weitestgehend entfernt werden. In einer Ausführungsform der vorliegenden Erfindung wird die Wundspüllösung nicht vollständig abgetrocknet, so dass eine Restdeuchte erhalten bleibt. Hierbei ist es erfindungsgemäß auch möglich, im darauf folgenden Schritt der Kaltplasmabehandlung neben der Wunde bzw. der wundnahen Bereiche auch die verbleibende Wundspüllösung mit dem Kaltplasma zu behandeln. Anschließend erfolgt erfindungsgemäß die Behandlung der Wunde und optional ggf. der genannten infizierten Bereiche in Wundnähe mit kaltem, atmosphärischem Plasma. Die Behandlung erfolgt dabei in Abhängigkeit der vorliegenden Wunde, wie für den Fachmann leicht ersichtlich. Relevante Parameter umfassen dabei Größe und Tiefe der Wunde, sowie ggf. Grad der Infektion. Eine typische Behandlungsdauer mit dem Kaltplasma kann dabei ebenfalls ca. 5 - 10 min, bevorzugt ca. 3 - 5 min betragen; bei größeren Wunden wie bspw. Sternumwunden in der Regel eher 5 - 10 min. Nach der Plasmabehandlung kann anschließend ein weiterer Schritt der Wundspülung erfolgen, analog zur ersten Wundspülung, wobei es sich bevorzugt in beiden Fällen um die gleiche Wundspüllösung handelt. In einer Ausführungsform der vorliegenden Erfindung erfolgt nach der Plasmabehandlung ein weiterer Schritt der Spülung mit einer Wundspüllösung, bevorzugt mit der gleichen Wundspüllösung wie bei der vorhergehenden Spülung. Danach kann erneut ggf. die Wundspüllösung mittels geeigneter Maßnahmen (bspw. Kompressen) weitestgehend entfernt werden.

In einer Ausführungsform der vorliegenden Erfindung kann auch die Wundspüllösung selbst mit Kaltplasma behandelt werden, bspw. ca. 5 - 10 min, oder ca. 3 - 5 min. Dies kann im Zusammenhang insbesondere dann der Fall sein, wenn die Wundspüllösung nach der Wundspülung nicht vollständig abgetrocknet wurde wie oben bereits beschrieben.

Nach dem Behandlung der Wunde mit dem Kaltplasma bzw. nach der anschließenden erneuten Spülung mit der Wundspüllösung wie im Rahmen der vorliegenden Erfindung dargestellt, wird die Wunde darüber hinaus mit einem Unterdruck-Aktivkohleverband (advanced Negative Pressure Wound Therapy (aNPWT)) zur Wundbehandlung behandelt,. Beispiele für geeignete Aktivkohleverbände sind dem Fachmann bekannt und umfassen u.a. Actisorb^{®}, Askina^{®} Carbosorb, CarboFlex^{®}, Vliwaktiv^{®} AG Saugkompresse, und andere. Als Unterdruck-Aktivkohleverband kann u.a. ein Verband aus Aktivkohle und Unterdruckschwamm verwendet werden, wie dem Fachmann bspw. als "advanced Negative Pressure Wound Therapy (aNPWT) bekannt ist. Die Aktivkohle unter dem Unterdruckschwamm kann residuale Keime fixieren und somit eine erneute Progression der Infektion reduzieren. Damit kann erfindungsgemäß der Behandlungsfortschritt durch die Kombination aus Wundspüllösung und kaltem atmosphärischem Plasma weiter unterstützt werden. Besonders bevorzugt ist bei der erfindungsgemäßen Anwendung ein Aktivkohleverband, bei dem der Aktivkohle enthaltende Teil direkt auf die Wunde aufgebracht (d.h. gelegt) wird. Dadurch können Bakterien ohne Barriere gebunden werden. Ein bevorzugter Aktivkohleverband, der ein direktes Aufbringen des Aktivkohle enthaltenden Teils des Verbands ermöglicht ist Zorflex^{®} Aktivkohle (Chemviron (England)).

Die Singularformen der Artikel der/die/das oder ein/eine umfassen im Zusammenhang der vorliegenden Erfindung stets auch die entsprechenden Pluralformen, soweit nicht explizit anders dargestellt.

Ferner umfasst der Ausdruck "und/oder" sowohl die jeweiligen Begriffe "und" und "oder", als auch alle oder jede mögliche Kombination der jeweiligen Elemente, die durch "und/oder" verbunden sind.

Der Begriff "etwa", "ca." oder "ungefähr" bedeutet bevorzugt innerhalb 20%, 10%, 5% oder 2% innerhalb des entsprechenden Wertes oder der Bandbreite. Darüber hinaus sind auch die exakten Werte oder Bandbreiten, die auf die Begriffe "etwa" oder "ungefähr" folgen, ausdrücklich umfasst.

Der Begriff "umfassend" wie hierin verwendet bedeutet die Einschließung der jeweiligen Merkmale, Begrifflichkeiten oder Werte, aber nicht die Exklusion nicht aufgeführter Merkmale, Begrifflichkeiten oder Werte. Eine Ausführungsform des Begriffs "umfassend" kann auch der ausschließende Begriff "bestehend aus" darstellen, sofern nicht explizit anders dargestellt.

Es sollte verstanden werden, dass diese Erfindung nicht auf die hierin beschriebenen speziellen Methoden, Protokolle, Material, Reagenzien und Substanzen usw. beschränkt ist und als solche variieren kann. Die hier verwendete Terminologie dient nur der Beschreibung bestimmter Ausführungsformen und soll den Umfang der vorliegenden Erfindung nicht einschränken, der ausschließlich durch die Ansprüche definiert ist.

Die folgenden Beispiele dienen lediglich der Illustration der vorliegenden Erfindung und schränken den Gegenstand der Erfindung in keiner Weise ein. Der Gegenstand der vorliegenden Erfindung wird durch die Ansprüche definiert.

### BEISPIELE

### Beispiel 1

### Allgemeines Behandlungsschema

Vor Therapiebeginn ist ggfs. die chirurgische Behandlung einer Wunde erforderlich. Der Wundbereich wird möglichst komplett eröffnet. Es wird ein allgemein-chirurgisches Debridement durchgeführt. Avitale Gewebe- und Knochenanteile werden bei Bedarf entfernt. Dies dient der ersten Reduzierung der Keimlast und der Vorbereitung des Wundgrundes. Nach Erreichen einer ausreichenden Hämostase kann ein erster Verband (Aktivkohle-NPWT) angelegt werden.

Die erfindungsgemäße Kombinationstherapie wird üblicherweise im Ambulanzbereich durchgeführt. Aufgrund des wenig schmerzhaften Procederes kann im Allgemeinen auf die Gabe von Analgetika oder gar auf ein Anästhesie-Standby verzichtet werden. Die Verbandswechsel werden unter sterilen Kautelen durchgeführt (sterile Handschuhe, Arbeitsflächen, Mundschutz). Aus diesem Grund sind zur sicheren und zügigen Durchführung dieser Therapie optimaler Weise zwei Personen erforderlich.

### Beispiel 2

### Spülung und Kaltplasma-Behandlung von Wunden

### Wundspülung zur Freisetzung von Singulett Sauerstoff in der Wunde

Nach Entfernen des OP-Verbandes (Fotodokumentation, Abstrichentnahme) erfolgt die Spülung des Wundgebietes mit einer Wundspüllösung mit aktiviertem Sauerstoff (z.B. ActiMaris, Kerrasol, Lavanox). Für den Fall, dass von außen Zugänge ins Körperinnere vorhanden sind, z.B. Driveline werden die zu behandelnden Abschnitte mit entsprechend getränkten Kompressen umwickelt. Die Einwirkzeit beträgt im Durchschnitt 5-8 min. Anschließend wird der Flüssigkeitsüberstand mit trockenen Kompressen entfernt, so dass im Wundbett ein feuchter Film der Spüllösung verbleibt.

### Kaltplasmabehandlung

Das zu behandelnde Wundareal wird mit Kaltplasma (z.B. Steriplas der Firma Adtec) therapiert. Aus Argongas wird in einem elektrischen Feld Argonplasma generiert, welches aus Luftsauerstoff wiederum die reaktiven Sauerstoffspezies (ROS) bildet. Diese bilden zusammen mit einem geringen Anteil an UV-Licht die wirksamen Bestandteile zur Bekämpfung der Krankheitserreger und zur Stimulation der körpereigenen Zellen zur besseren Infektabwehr. Neben der direkten Plasmawirkung (hohe Eindringtiefe aufgrund atomarer Struktur) wird über die Reste der Spülflüssigkeit eine Verlängerung der sog. Plasmawirkzeit erreicht. Die Einwirkzeit des Kaltplasmas beträgt pro behandelten Flächenabschnitt ca. 5 min (d.h. bei offenem Sternum z.B. 8 - 10 min).

### Beispiel 3

### Wundspülung mit aktiviertem Sauerstoff, Kaltplasmabehandlung und Wundversiegelung mit Aktivkohle-NPWT

### Erneute Wundspülung mit Freisetzung von Singulett Sauerstoff

Direkt im Anschluss an die Kaltplasmabehandlung wird der Wundgrund wieder möglichst vollständig mit der Spüllösung behandelt (ggfs. wiederum feuchte Kompressen für Zugänge von außen, z.B. Driveline). Dies erfolgt zum einen zur Ausnutzung der zur Verfügung stehenden Behandlungszeit, aber auch, um den Wundgrund vor Verunreinigungen zu schützen bis der versiegelnde Verband aufgebracht ist. Die Einwirkzeit, welche ebenfalls nochmals ca. 5 min beträgt, wird genutzt um im letzten Behandlungsschritt den Aktivkohle-NPWT Verband vorzubereiten. Unmittelbar vor Einlegen der Aktivkohlefolie wird die Flüssigkeit wieder mit trockenen Kompressen entfernt.

### Anlegen des Aktivkohle-NPWT Verbandes (aNPWT)

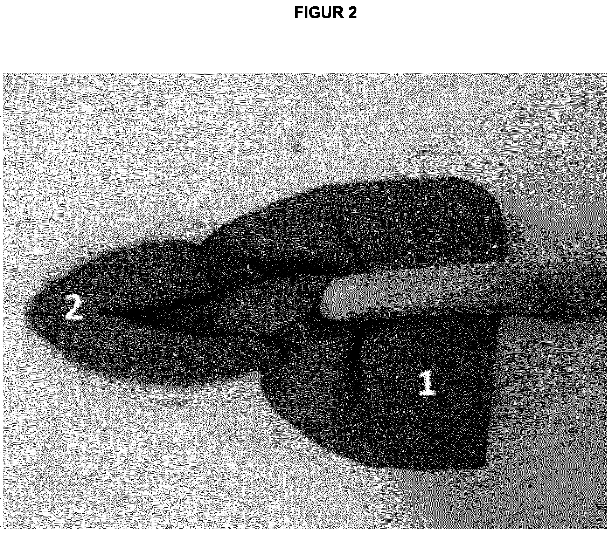

Auf einer sterilen Unterlage wird der NPWT Schwamm zugeschnitten. Die Aktivkohlefolie wird im Wundgrund platziert. Hierbei ist zu beachten, dass die Folie nach Möglichkeit den kompletten Wundgrund ausfüllt (ein- oder zweilagig), um einen bestmöglich-flächigen Kontakt zum Wundgewebe herzustellen. Die Kohlefolie sollte aus der Wunde herausragen, um das umliegende Hautareal noch für 1 bis 2 cm zu überdecken (Wundrandschutz zur Verhinderung der Keimeinwanderung). Anschließend wird der passend zugeschnittene NPWT-Schwamm eingebracht und entsprechend mit einer Versiegelungsfolie abgeklebt **(****Figur 2****).** Bei tiefen sternalen Infekten ist auch ein doppellagiges Aufbringen von Folie und Schwamm möglich: Erst Lage Aktivkohle zum Schutz des Herzens, dann intersternaler Schwamm, dann Aktivkohle für das prästernale Weichteilgewebe und zum Schluss ein oberflächlicher Schwamm.

### Wechselintervalle

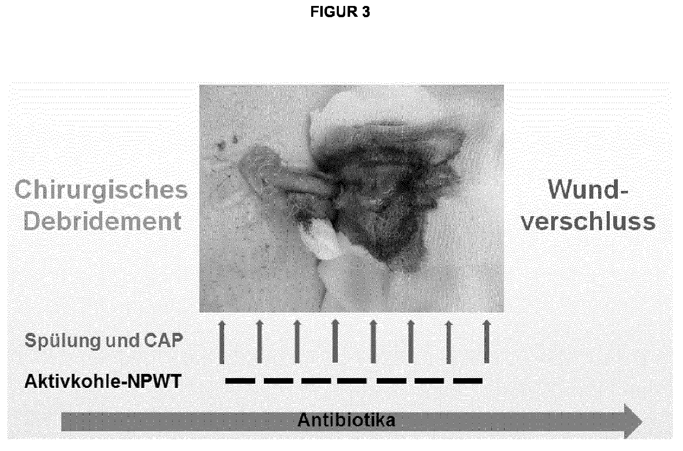

Ziele der intensivierten Wundbehandlung ist eine Verkürzung der Therapiezeit, welche letztlich das Risiko für die Patienten (Wundkomplikationen) senken soll und im Langzeitverlauf Kosten einsparen helfen. Aus diesen Gründen ist es erforderlich die Wechselintervalle **(****Figur 3****)** zu verkürzen. Waren bei der bisher üblichen Behandlung mit Standard-NPWT Zeitintervalle von 4 bis 5 Tagen normal, so sollten unter diesem Behandlungsregime nach Möglichkeit alle 2 bis 3 Tage ein Verbandswechsel mit Kaltplasmaapplikation durchgeführt werden.

Die verkürzten Wechselintervalle verhindern, dass eine Regeneration der Krankheitserreger in der NPWT-Zeit deutlich erschwert wird, zumal bei jeder Behandlung frische Wirksubstanzen (insbesondere der Singulett Sauerstoff) aufgebracht werden. Die Aktivkohlefolie in der versiegelten Wunde hemmt die Migration der Krankheitserreger und hilft so, mögliche residuelle Infektherde schneller einzugrenzen.

### Abschluss

Nach Konsolidierung des Wundgrundes (Ergebnis Abstrichuntersuchung, Entzündungswerte (z.B.: CRP-Verlauf), makroskopischer Befund, Granulation) erfolgt der operative Wundverschluss. Auch im OP kann optional nochmals mit Spüllösung und Kaltplasma behandelt werden.

### Beispiel 4

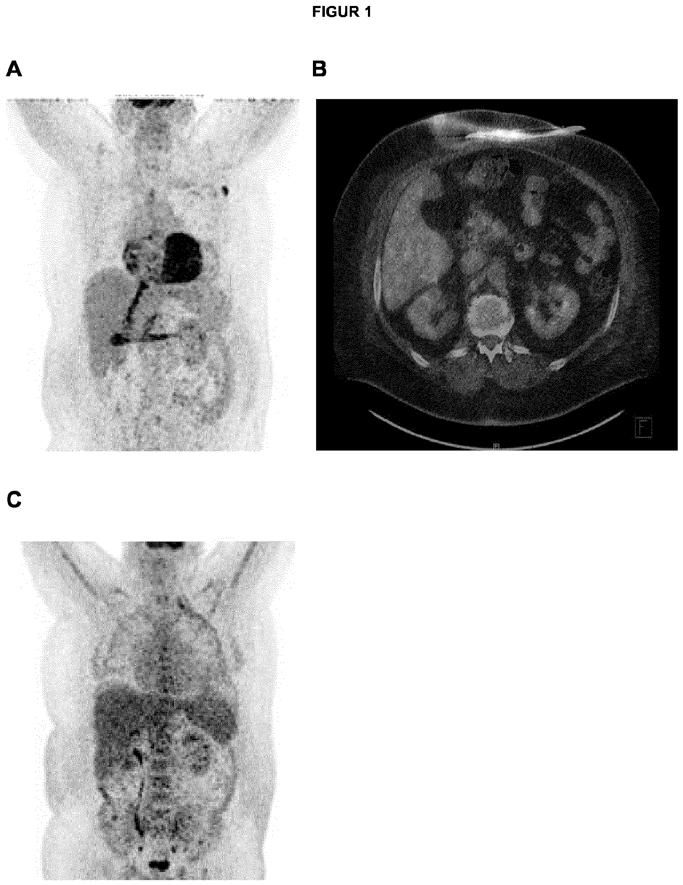

Der Patient erhielt 2013 ein Kunstherz (LVAD). Seit Herbst 2015 befand er sich wegen Drivelineinfektion zunächst in ambulanter Behandlung. Es erfolgte eine erste dokumentierte Behandlung der Driveline mit ActiMaris^{®}. Einige Monate später erfolgte die stationäre Aufnahme zur operativen Sanierung des Infektproblems. Der Befund war so ausgeprägt, dass der Patient aufgrund des Pseudomonasbefalls bereits roch, bevor er durch die Tür eintrat. Der Grad der Infektion ist in den **Figuren 1A** und **1B** gezeigt. Die operative Revision erfolgt unmittelbar. Nach Abklingen der Blutungsneigung wurde der Patient mit dem erfindungsgemäßen Kombinationsverfahren behandelt. Innerhalb von nur drei Wochen normalisierten sich die Infektparameter (CRP < 0,5) (siehe **Figur 1C****)** und der Patient wurde auf eigenen Wunsch mit der Wunde in die ambulante Betreuung entlassen.

Anzumerken ist, dass die alleinige Behandlung mit ActiMaris^{®} über ein halbes Jahr nicht erfolgreich war, so dass es zu einem deutlichen Progress der Infektion mit Anstieg der Infektparameter kam. Nach dreiwöchiger Kombinationstherapie konnten oben gezeigte Befundbesserung mit Normalisierung der Infektwerte erreicht werden.

## Patentansprüche

1. Kombination von Wundspüllösung, kaltem atmosphärischem Plasma und einem Aktivkohleverband zur Verwendung in einem Verfahren bei der Behandlung von Wunden, umfassend folgende Schritte:
(a) Spülen der Wunde mit einer Wundspüllösung,
(b) Behandeln der Wunde mit kaltem atmosphärischem Plasma,
(c) Anlegen eines Unterdruck-Aktivkohleverbandes auf die Wunde.

2. Kombination zur Verwendung nach Anspruch 1, ferner umfassend
(d) Erneutes Spülen der Wunde mit einer Wundspüllösung.

3. Kombination zur Verwendung nach einem der Ansprüche 1 bis 2, ferner umfassend Behandeln der auf der Wunde verbliebenen Wundspüllösung mit kaltem atmosphärischen Plasma.

4. Kombination zur Verwendung nach einem der Ansprüche 1 bis 3, wobei die Wundspüllösung reaktiven Sauerstoff enthält.

5. Kombination zur Verwendung nach einem der Ansprüche 1 bis 4, wobei die Wundspüllösung Singulett-Sauerstoff enthält.

6. Kombination zur Verwendung nach einem der Ansprüche 1 bis 5, wobei zur Plasmaerzeugung Umgebungsluft oder Argon eingesetzt wird.

7. Kombination zur Verwendung nach einem der Ansprüche 1 bis 6, wobei der Salzgehalt der Wundspüllösung unter 4 Gew% liegt.

8. Kombination zur Verwendung nach einem der Ansprüche 1 bis 7, wobei der Aktivkohle enthaltende Teil des Aktivkohleverbands direkt auf die Wunde aufgebracht wird.

## Claims

1. A combination of a wound rinsing solution, a cold atmospheric plasma and an activated carbon dressing for use in a method for the treatment of wounds, comprising the following steps:
(a) rinsing the wound with a wound rinsing solution,
(b) treating the wound with cold atmospheric plasma,
(c) applying a vacuum activated carbon dressing to the wound.

2. The combination for the use according to claim 1, further comprising
(d) rinsing the wound again with a wound rinsing solution.

3. The combination for the use according to any one of claims 1 to 2, further comprising treating the wound rinsing solution remaining on the wound with cold atmospheric plasma.

4. The combination for the use according to any one of claims 1 to 3, wherein the wound rinsing solution contains reactive oxygen.

5. The combination for the use according to any one of claims 1 to 4, wherein the wound rinsing solution contains singlet oxygen.

6. The combination for the use according to any one of claims 1 to 5, wherein ambient air or argon is used for plasma generation.

7. The combination for the use according to any one of claims 1 to 6, wherein the salt content of the wound rinsing solution is less than 4% by weight.

8. The combination for the use according to any one of claims 1 to 7, wherein the part of the activated carbon dressing containing activated carbon is applied directly to the wound.

## Revendications

1. Combinaison d'une solution de rinçage des plaies, d'un plasma atmosphérique froid et d'un pansement au charbon actif, destinée à être utilisée dans une méthode de traitement des plaies, comprenant les étapes suivantes
a) rincer la plaie avec une solution de rinçage des plaies,
b) traiter la plaie avec du plasma atmosphérique froid,
c) application d'un pansement au charbon actif à pression négative sur la plaie.

2. Combinaison pour utilisation selon la revendication 1, comprenant en outre
d) rincer à nouveau la plaie avec une solution de rinçage des plaies.

3. Combinaison destinée à être utilisée selon l'une quelconque des revendications 1 à 2, comprenant en outre le traitement de la solution de rinçage de la plaie restant sur la plaie avec un plasma atmosphérique froid.

4. Combinaison destinée à être utilisée selon l'une quelconque des revendications 1 à 3, dans laquelle la solution de rinçage des plaies contient de l'oxygène réactif.

5. Combinaison destinée à être utilisée selon l'une quelconque des revendications 1 à 4, dans laquelle la solution de rinçage des plaies contient de l'oxygène singlet.

6. Combinaison destinée à être utilisée selon l'une quelconque des revendications 1 à 5, dans laquelle on utilise de l'air ambiant ou de l'argon pour générer le plasma.

7. Combinaison destinée à être utilisée selon l'une quelconque des revendications 1 à 6, dans laquelle la teneur en sel de la solution de rinçage des plaies est inférieure à 4 % en poids.

8. Combinaison destinée à être utilisée selon l'une quelconque des revendications 1 à 7, dans laquelle la partie du pansement au charbon actif contenant du charbon actif est appliquée directement sur la plaie.
